# DEMANDE DE BREVET EUROPEEN

(11) **EP 3 167 852 A1**
(43) Date de publication de la demande: **17.05.2017**
(21) Numéro de dépôt: 15194779.3
(22) Date de dépôt: 16.11.2015
(51) Int. Cl.: A61F 5/56

(54) **GOUTTIÈRE DENTAIRE, PROCÉDÉ DE RÉALISATION ET ORTHÈSE COMPRENANT UNE TELLE GOUTTIÈRE**

(71) Demandeur: Oscimed SA, 2300 La Chaux-de-Fonds (CH)
(72) Inventeur: Magnin, Jacques, 2300 La Chaux-de-Fonds (CH)
(74) Mandataire: Gevers SA

(57) **Abrégé**

La présente invention concerne une gouttière dentaire (1), comprenant une armature (2) d'une matière synthétique rigide supportant une coque (3) de matière thermoformable, ladite armature (2) comportant une paroi antérieure, dite paroi labiale (21), et une paroi postérieure, dite paroi linguale (22), sensiblement parallèles l'une de l'autre et reliées par une paroi de fond, dite paroi occlusale (23), sensiblement perpendiculaire aux deux parois labiale (21) et linguale (22) de sorte que l'armature (2) présente dans tout plan vertical une section en U, caractérisée en ce que ladite coque thermoformable (3) est logée entre lesdites parois labiale (21), linguale (22) et occlusale (23) de ladite armature essentiellement et ancrée sur celle-ci par l'intermédiaire de moyens d'ancrage (4, 5) agencés dans l'armature (2) et la coque thermoformable (3).

## Description

### Domaine technique

La présente invention se rapporte au domaine de l'appareillage buccal pour le traitement et/ou la prophylaxie de pathologies dentaires, mandibulaires et/ou respiratoires chez l'humain. Elle concerne, plus particulièrement, une gouttière dentaire d'adaptabilité améliorée et d'encombrement réduit dans la bouche des patients.

### Etat de la technique

Les gouttières dentaires (occlusales et mandibulaires) ainsi que les dispositifs comprenant de telles gouttières appartiennent à l'ensemble des orthèses orales qui comprend également les protège-dents, les appareillages de traitement des apnées du sommeil, et les dispositifs intra-oraux orthopédiques adaptés au traitement des Dysfonctionnements de l'Appareil Manducateur (DAM), tel le bruxisme par exemple, mais également d'autres indications respiratoires telles le ronflement et l'apnée du sommeil.

Le bruxisme est une pathologie commune plus généralement connue sous le nom de « grincement des dents » qui consiste en un frottement et/ou un serrement intempestif des dents en dehors des périodes de mastication ou de déglutition, durant lesquelles les dents se touchent naturellement. Ces phases de grincement ou de serrement peuvent être diurnes et/ou nocturnes. Si les phases diurnes ne posent généralement pas de problèmes et sont liées aux mécanismes naturels de relâchement des mâchoires, les phases nocturnes, incontrôlées, peuvent engendrer des conséquences diverses plus ou moins néfastes ou pénalisantes pour les individus sujets de cette pathologie telles que :
- usure de l'émail et, le cas échéant de la dentine (couche d'ivoire sous-jacente),
- sensibilisation accrue des dents aux variations de températures et d'acidité des aliments et liquides ingérés,
- fractures dentaires,
- perturbations posturales.

La pose d'une gouttière dentaire occlusale constitue à ce jour le traitement privilégié du bruxisme, visant essentiellement à prévenir et limiter notamment l'usure des dents.

Le ronflement, par ailleurs appelé ronchopathie, est une pathologie de l'appareil respiratoire liée au relâchement musculaire et tissulaire au niveau de la mandibule et à l'intérieur de la bouche pendant le sommeil. Ce relâchement provoque un recul de la langue vers l'entrée de la trachée qui engendre des perturbations du flux respiratoire et des phénomènes de vibration de la luette et des tissus mous du palais à l'origine d'un bruit, caractéristique du ronflement.

Le ronflement, dans une majorité de cas, constitue une perturbation bénigne de la respiration et du sommeil des individus qui y sont sujets, voire de leurs proches lorsque les niveaux acoustiques, qui peuvent atteindre 100 dB, sont élevés. Cependant, il peut également chez certains individus être source de plus importantes perturbations et notamment d'interruptions respiratoires qui perturbent le sommeil. On parle dans ce cas d'apnée du sommeil. L'apnée engendre usuellement une fatigue et une somnolence diurne, ainsi que des risques accrus d'hypertension et de maladies cardiovasculaires telles que, notamment, des accidents vasculaires cérébraux et infarctus.

Pour lutter contre le ronflement et l'apnée du sommeil ont été proposés des dispositifs intra-oraux comportant au moins une gouttière mandibulaire configurée pour promouvoir un déplacement vers l'avant (postéro-antérieur) de la mandibule pendant le sommeil, de manière à compenser le relâchement musculaire et limiter le recul de la langue vers le pharynx et la trachée, cause principale de l'obstruction respiratoire source de ronflement et d'apnée durant le sommeil.

Différentes configurations de gouttières occlusales et/ou mandibulaires ainsi que d'orthèses comportant de telles gouttières ont été proposées dans l'art antérieur, tel que par exemple dans les demandes de brevet EP 1 602 347 A1 et EP 1 750 629 A2.

Ces demandes décrivent notamment des orthèses d'avancement mandibulaire comprenant deux gouttières adaptées à l'arcade dentaire supérieure et l'arcade dentaire inférieure de la mâchoire et reliées entre elles de manière à promouvoir un mouvement de la gouttière inférieure et de la mandibule par appui de la gouttière sur l'arcade dentaire correspondante dans le sens postéro-antérieur. Les gouttières de ces orthèses comprennent d'une part une coque thermoformable à la configuration particulière des arcades et implantations dentaires de l'utilisateur de l'orthèse ainsi qu'une armature d'une matière sensiblement rigide supportant ou surmoulée dans la coque thermoformable. L'armature rigide des gouttières présente une forme générale en U conforme à une arcade dentaire et comporte des parois antérieure et postérieure reliées à leur base par une paroi de fond conférant ainsi à l'armature une section verticale également en U de telle sorte que ladite armature (et la coque thermoformable) puissent recouvrir et s'emboiter sur chacune des arcades dentaires en recouvrant les faces postérieures et antérieures de celles-ci ainsi que le plan d'occlusion de chacune d'elles. L'armature rigide des gouttières est par ailleurs articulée par le biais de fentes ménagées symétriquement dans ses parois antérieures et postérieures ainsi qu'au moins partiellement dans sa paroi de fond. Ces fentes autorisent une déformation de l'armature rigide concomitamment à la coque thermoformable de chacune des gouttières pour s'adapter à la conformation dentaire. Il en résulte des gouttières et orthèses productibles en grande série mais conformables individuellement à la configuration particulière des arcades dentaires de chaque patient, pour un confort et une efficacité accrus de l'orthèse et de ses gouttières.

Cependant, les gouttières connues de l'art antérieur, bien qu'adaptables à la conformation particulière de chacune des arcades dentaires d'un patient par le caractère thermoformable de leur coque et le caractère articulé de leur armature, ne sont pas entièrement satisfaisantes sur le plan du confort d'utilisation pour les patients. En effet, ces gouttières présentent un encombrement important au niveau des arcades dentaires, notamment en raison du surmoulage complet de l'armature dans la coque thermoformable, qui engendre un gêne pour l'utilisateur aussi bien sur les faces antérieures labiales des arcades dentaires, entre les dents et les lèvres, que sur les faces postérieures linguales des arcades dentaires entre les dents et le palais ou la langue.

Un but de la présente invention est par conséquent de proposer une gouttière dentaire exempte des inconvénients de l'art antérieur.

Un autre but de la présente invention est de proposer un kit et un procédé de mise en forme d'une telle gouttière dentaire permettant à tout patient de réaliser lui-même une gouttière selon l'invention adaptée à la conformation individuelle d'une de ses arcades dentaires.

Un autre but de l'invention consiste enfin à proposer une orthèse intégrant une gouttière conforme à la présente invention.

### Divulguation de l'invention

Ces buts sont atteints selon un objet de l'invention par une gouttière dentaire comprenant une armature d'une matière synthétique rigide supportant une coque de matière thermoformable, ladite armature comportant une paroi antérieure, dite paroi labiale, et une paroi postérieure, dite paroi linguale, sensiblement parallèles l'une de l'autre et reliées par une paroi de fond, dite paroi occlusale, sensiblement perpendiculaire aux deux parois labiale et linguale de sorte que l'armature présente dans tout plan vertical une section en U, caractérisée en ce que ladite coque thermoformable est logée entre lesdites parois labiale, linguale et occlusale de ladite armature essentiellement et ancrée sur celle-ci par l'intermédiaire de moyens d'ancrage agencés dans l'armature et la coque thermoformable.

La gouttière dentaire selon l'invention est ainsi agencée de telle sorte que la coque thermoformable de celle-ci soit essentiellement reçue entre les parois de l'armature de la gouttière et non majoritairement en surmoulage de celle-ci comme dans l'art antérieur. En effet, un surmoulage complet de l'armature par la coque thermoformable n'est pas nécessaire en soi à la fonctionnalité de la gouttière mais augmente grandement son encombrement dans la bouche des patients.

De plus la gouttière de l'invention compense avantageusement les risques de déchaussement de la coque thermoformable de l'armature par la présence de moyens d'ancrage de la coque sur l'armature, moyens d'ancrage opérant une liaison localisée de la coque thermoformable sur l'armature, en des positions choisies pour perturber a minima le positionnement de la gouttière dans la bouche et réduire au maximum son encombrement.

Selon un mode de réalisation de la gouttière de l'invention, les moyens d'ancrage comportent au moins une patte de liaison recouvrant au moins partiellement au moins un segment de la paroi labiale et/ou de la paroi linguale, ladite patte de liaison étant formée de matière avec la coque thermoformable de manière à pincer ledit au moins un segment de paroi labiale et/ou linguale entre la coque thermoformable et la patte de liaison.

Selon un mode de réalisation, les moyens d'ancrage comportent des orifices formés de part en part entre une surface interne de la paroi occlusale sur laquelle repose la coque thermoformable, et une surface externe de la paroi occlusale définissant un plan d'occlusion de la paroi occlusale, lesdits orifices présentant au moins une dimension, prise dans au moins une section verticale de la paroi occlusale, qui est inférieure au niveau de la surface interne par rapport à la dimension correspondante au niveau de la surface externe.

Cette configuration particulière des orifices assure la formation de tenons de matière thermoformable de la coque thermoformable au travers des orifices, lesdits tenons ne pouvant se retirer vers la coque une fois insérés dans les orifices du fait de l'expansion de la matière thermoformable de la coque vers la surface externe de l'armature, dans la plus grande dimension des orifices.

De préférence, les dits orifices présentent une dimension variable de manière croissante sur l'épaisseur de la paroi occlusale entre la surface interne et la surface externe de ladite paroi.

De préférence, selon ce mode particulier de réalisation, lesdits orifices ont une section, prise dans ladite section verticale de la paroi occlusale, au moins partiellement triangulaire ou trapézoïdale.

Dans un mode de réalisation de l'invention, ladite armature est flexible de part et d'autre d'au moins un plan vertical perpendiculaire de ses parois labiale, linguale et occlusale.

Le cas échéant, dans une forme de réalisation, ladite armature est flexible de part et d'autre d'une pluralité de plans verticaux perpendiculaires à sa paroi occlusale.

Dans un mode de réalisation de l'invention, ladite armature comporte au moins une zone de fléchissement privilégiée formée par réduction localisée de la matière constitutive de l'armature.

De préférence, ladite au moins une zone de fléchissement privilégiée s'étend dans au moins un dit plan vertical de part et d'autre duquel ladite armature est flexible.

De préférence encore, la au moins une zone de fléchissement privilégiée comporte un évidement, en particulier une fente, formée dans au moins une paroi labiale ou linguale et la paroi occlusale de l'armature.

Selon une forme de réalisation avantageuse, la au moins une zone de fléchissement privilégiée comporte un évidement formé exclusivement dans la paroi linguale et la paroi occlusale.

Selon une autre forme de réalisation avantageuse, la gouttière de l'invention comporte plusieurs zones de fléchissement formées par des évidements ou retraits de matière localisés dans les parois linguale et occlusale.

Selon un second objet, la présente invention concerne un procédé d'ajustement d'une gouttière dentaire telle que précédemment décrite, comprenant les étapes suivantes :
- Chauffage de la gouttière dentaire dans un bain-marie à une température comprise entre 60°C et 90°C pour ramollir la coque de matière thermoformable pendant une durée de 1 min au moins;
- Positionnement de la gouttière dentaire sur une plaque occlusale conformée sensiblement en Y, la plaque occlusale comportant une branche de saisie manuelle et deux branches de support de la gouttière dentaire jointes à une de leurs extrémités et à une extrémité de la branche de saisie manuelle ainsi que des plots de calage de ladite gouttière ajustés sur une face de ladite plaque occlusale au moins ;
- Insertion de la plaque occlusale portant la gouttière dentaire dans la bouche d'un patient,
- Mise en forme de la coque thermoformable par pression d'une arcade dentaire du patient dans la coque thermoformable ramollie et contrepression sur la plaque occlusale de l'arcade dentaire opposée de manière à procurer un effet de coffrage de la matière ramollie de la coque dans l'armature;
- Retrait de la plaque occlusale et de la gouttière dentaire de la bouche du patient.

Le procédé de l'invention permet par la mise en oeuvre d'une plaque occlusale en support de la gouttière de la présente invention de permettre une prise d'empreinte dentaire et une mise en forme de la gouttière extrêmement simple et mais tout en maitrisant la fuite de matière thermoformable au travers des différents orifices ou fentes pratiqués dans l'armature de la gouttière de l'invention. En effet, la plaque occlusale procure lors de la prise d'empreinte une surface de coffrage ou de repoussement de la matière de la coque thermoformable pour contraindre celle-ci dans entre les parois de l'armature sans débordements sur l'extérieur des parois de l'armature.

Le cas échéant, le procédé de l'invention peut en outre comporter une étape additionnelle de refroidissement de la gouttière dentaire après retrait de la plaque occlusale de la bouche du patient.

Dans un troisième objet, la présente invention concerne également un kit de mise en oeuvre d'un procédé tel que définit ci-dessus, comportant une plaque occlusale comportant une branche de saisie manuelle et deux branches de support de la gouttière dentaire jointes à une de leurs extrémités et à une extrémité de la branche de saisie manuelle ainsi que des plots de calage de ladite gouttière ajustés sur une face de ladite plaque occlusale au moins et une gouttière dentaire selon l'invention. L'association d'une gouttière conforme à l'invention et d'une plaque occlusale adaptée pour supporter ladite gouttière lors de la mise en forme de celle-ci permet une mise en oeuvre aisée pour le patient du procédé de mis en forme décrit précédemment, sans avoir recours à un tierce équipement.

Selon un quatrième objet, l'invention propose également une orthèse mandibulaire comportant au moins une gouttière dentaire telle que définie précédemment.

### Brève description des dessins

D'autres détails de l'invention apparaîtront plus clairement à la lecture de la description qui suit, faite en référence au dessin annexé dans lequel :
- La figure 1 représente une vue d'ensemble d'une gouttière dentaire selon l'invention ;
- La figure 2 représente une vue de droite de la gouttière de la figure 1 ;
- La figure 3 représente une vue de la face antérieure (labiale) de la gouttière de la figure 1 ;
- La figure 4 représente une vue de la face postérieure (linguale) de la gouttière de la figure 1 ;
- La figure 5 représente une vue de dessus de la de la gouttière de la figure 1 ;
- La figure 6 représente une coupe verticale A-A selon un plan sagittal M de la gouttière de la figure 3 ou la figure 4 ;
- La figure 7 représente une coupe verticale B-B suivant un plan vertical B représenté sur la figure 5 ;
- La figure 8 représente une vue d'ensemble d'un kit pour réaliser une gouttière telle que représentée à la figure 1 ;
- Les figures 9 à 12 représentent différentes vues d'une orthèse mandibulaire comportant au moins une gouttière dentaire selon l'invention.

### Mode de réalisation de l'invention

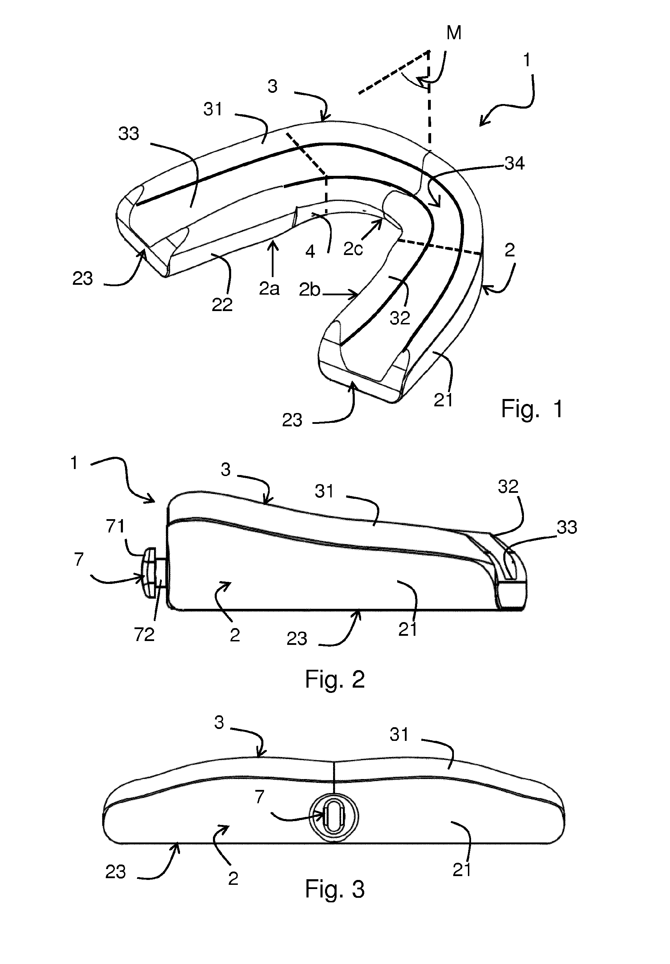

Les figures 1 à 7 représentent une gouttière dentaire 1 selon un mode de réalisation de l'invention sous différentes vues de celle-ci.

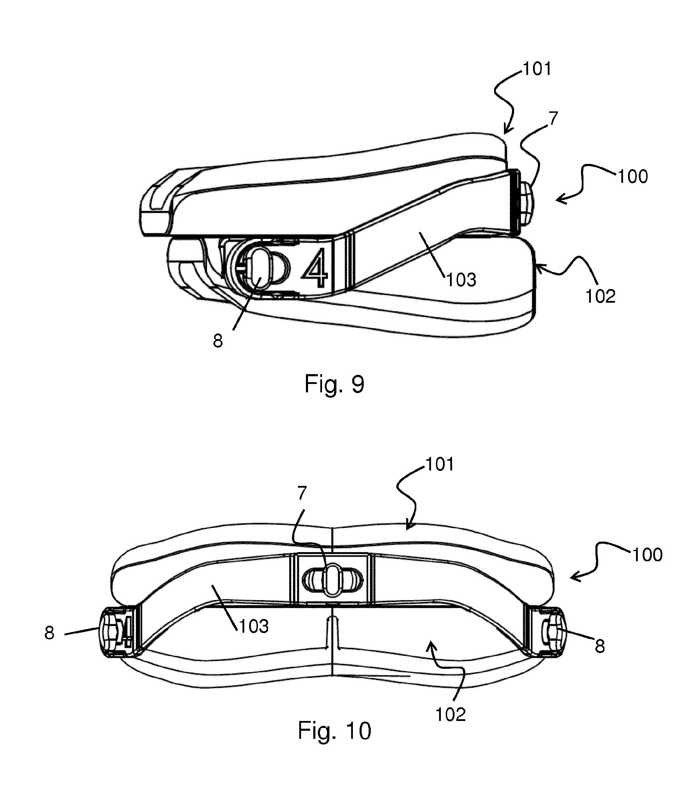

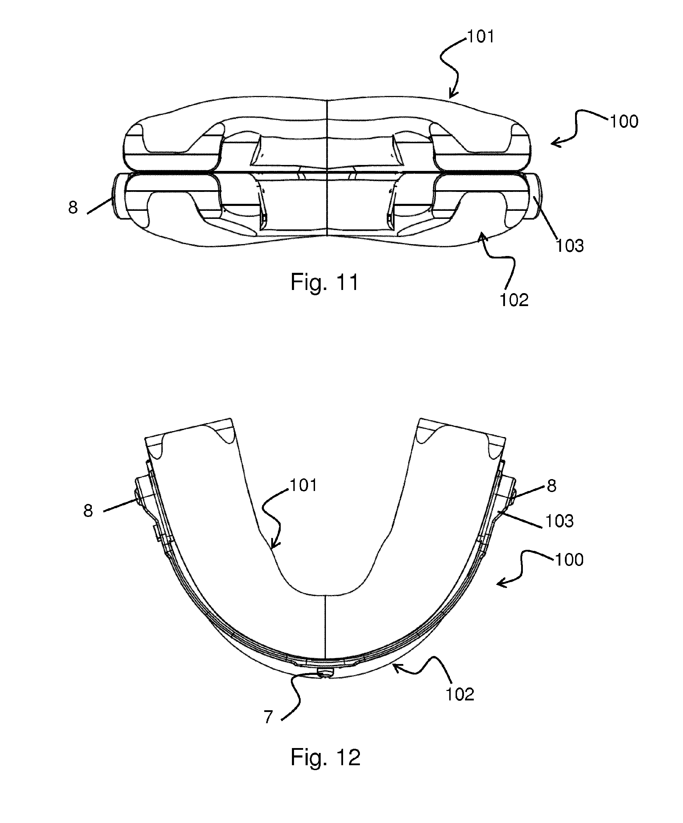

Une telle gouttière dentaire 1 peut avantageusement être utilisée en tant que gouttière occlusale et/ou comme gouttière mandibulaire, le cas échéant dans le cadre d'une orthèse mandibulaire comme représenté aux figures 9 à 12.

Comme représenté aux figures 1 à 7, la gouttière dentaire 1 de l'invention se compose de façon classique d'une armature 2 et d'au moins une coque 3 thermoformable supportée et maintenue sur l'armature 2.

L'armature 2 est de préférence constituée d'une matière synthétique biocompatible et/ou à tout le moins de qualité alimentaire sensiblement rigide. En particulier, l'armature 2 peut être constituée de polyéther block amide ou de polyester par exemple et formée par moulage ou injection.

De façon classique l'armature 2 présente une forme analogue à celle d'une arcade dentaire, c'est-à-dire une forme sensiblement en U et comporte deux sections latérales 2a, 2b droites reliées entre elles par une section centrale 2c incurvée. Les sections droites 2a, 2b sont ainsi conformées pour, lors d'une utilisation normale de la gouttière dentaire 1, recouvrir les champs latéraux postérieurs d'une arcade dentaire d'un patient humain comportant les prémolaires et molaires quand la section centrale incurvée est destinée à recouvrir le champ frontal de l'arcade dentaire et les incisives et canines du patient, en considérant une denture complète et régulière bien entendu.

L'armature 2 présente en outre, de façon là aussi classique, une paroi antérieure sensiblement verticale, dite paroi labiale 21 et une paroi postérieure verticale, dite paroi linguale 22. Les parois labiale 21 et linguale 22 sont reliées entre elles par une troisième paroi de l'armature, à savoir une paroi de fond, dite paroi occlusale 23, s'étendant perpendiculairement aux parois labiale 21 et linguale 22 et les reliant à leur base.

On comprend ici que la paroi labiale 21 est une paroi de l'armature 2 de la gouttière dentaire 1 qui, dans l'utilisation normale de ladite gouttière dentaire 1 est localisée entre l'arcade dentaire du patient un une lèvre de celui-ci. De la même façon, on comprend par paroi linguale 22 une paroi de l'armature 2 de la gouttière dentaire 1 qui, dans l'utilisation normale de ladite gouttière dentaire 1 est localisée entre l'arcade dentaire du patient et la langue ou le palais de celui-ci. On comprend enfin par paroi occlusale 23 une paroi de l'armature 2 de la gouttière dentaire 1 qui, dans l'utilisation normale de ladite gouttière dentaire 1 est localisée dans et/ ou définit un plan d'occlusion entre les dents du patient, ou à tout le moins une paroi délimitant une interface de contact entre les dents des arcades dentaires du patient.

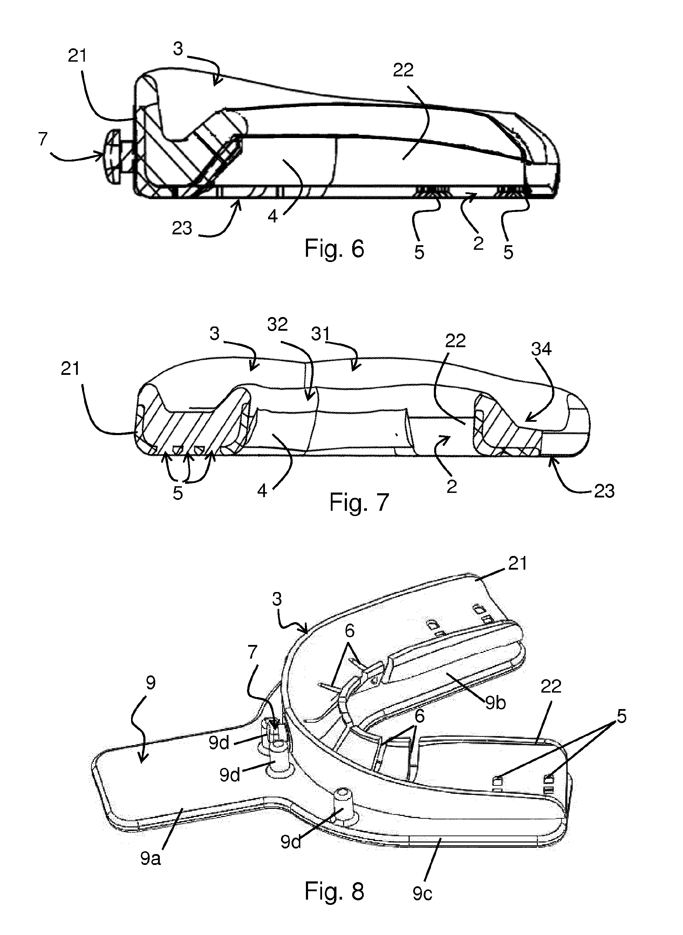

Ainsi, les parois labiale 21 et linguale 22 sont sensiblement parallèle l'une de l'autre et sensiblement perpendiculaire à la paroi occlusale 23 de sorte que l'armature 2 présente dans tout plan vertical une section sensiblement en U, comme cela ressort en particulier de la figure 6.

La coque 3 de la gouttière dentaire 1 est positionnée sur l'armature 2, en particulier logée ou insérée entre les parois labiale 21, linguale 22 et occlusale 23 essentiellement. En d'autres termes, l'armature 2 et ses parois 21, 22, 23 forment un moule de support et de mise en forme de la coque 3 thermoformable de la gouttière dentaire 1.

La coque 3 thermoformable est constituée d'une matière thermoplastique de qualité alimentaire et/ou biocompatible. De préférence, la coque 3 thermoformable est réalisée en éthylène-acétate de vinyle, plus généralement connu sous l'acronyme EVA, de l'anglais ethylen-vinyl acetate.

La coque 3 thermoformable épouse la forme de l'armature 2 rigide et présente par conséquent une forme en U analogue à celle de l'armature 2. La coque 3 thermoformable épousant les parois 21, 22, 23 de l'armature 2 forme une goulotte de recouvrement d'une arcade dentaire d'un patient qui comporte une paroi labiale 31 reposant contre une surface interne de la paroi labiale 21 de l'armature, une paroi linguale 32 reposant contre une surface interne de la paroi linguale 22 de l'armature 2 et une paroi de fond 33 reposant contre la surface interne de la paroi occlusale 23 de l'armature 2.

Ainsi, les parois labiales 21, 31 et linguales 22, 32 sont sensiblement parallèles les unes des autres et sensiblement perpendiculaires à la paroi occlusale 23 et la paroi de fond 33 de la coque 3 thermoformable de sorte que la gouttière 1 dans son ensemble, comprenant l'armature 2 et la coque 3 thermoformable reçue sur celle-ci présente dans tout plan vertical une section sensiblement en U, comme cela ressort des figures 1 à 7.

De préférence, pour procurer un meilleur confort d'utilisation de la gouttière dentaire 1, les parois labiale 31 et linguale 32 de la coque 3 thermoformable s'étendent en saillie au-dessus du bord supérieur des parois labiale 21 et linguale 22 de l'armature de manière à former un bourrelet d'appui de la coque 3 thermoformable sur l'armature 2 apte à procurer une surface de contact mousse avec les tissus buccaux d'un patient lors de l'utilisation de la gouttière dentaire 1.

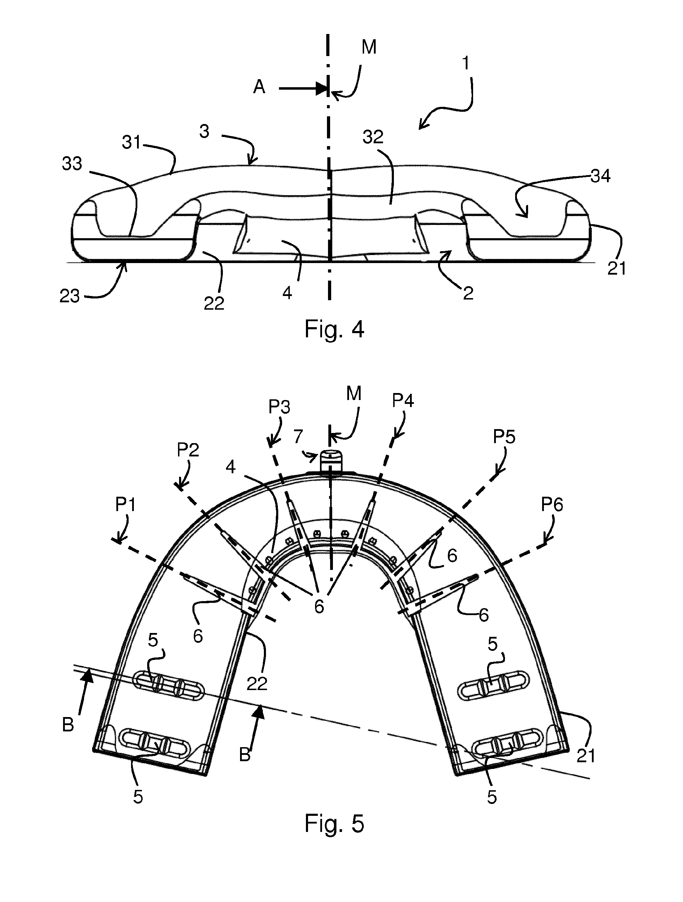

Conformément à l'invention et comme cela ressort des figures 4 à 7 en particulier, la coque 3 thermoformable de la gouttière dentaire 1 est ancrée sur l'armature par l'intermédiaire de moyens d'ancrage agencés dans l'armature 2 et la coque 3. En particulier les moyens d'ancrage comportent en premier lieu au moins une patte de liaison 4 formée de matière avec la coque 3 de matière thermoformable, en extension de la paroi linguale 32 de celle-ci sur au moins une portion ou segment de la paroi linguale 22 de l'armature 2, par exemple par surmoulage de la matière constitutive de la coque 3 thermoformable sur la paroi linguale de l'armature 2.

Bien entendu, il est également envisageable dans le cadre de l'invention de réaliser la patte de liaison 4 non pas sur la paroi linguale 22 mais sur une portion ou segment de la paroi labiale 21 de l'armature 2 à partir de la paroi labiale 31 de la coque 3 de matière thermoformable. Il est également possible enfin de réaliser plusieurs pattes de liaison 4 recouvrant au moins partiellement au moins un segment de la paroi labiale 21 et de la paroi linguale 22 de l'armature pour réaliser plusieurs points d'ancrage de la coque 3 thermoformable le long des parois 21 ,22 de l'armature 2.

La réalisation d'une patte de liaison 4 telle que décrit ci-avant permet de réaliser un ancrage de la coque 3 thermoformable sur l'armature 2 par effet de pincement de la coque sur la paroi labiale 21 et/ou linguale 22 de l'armature 2 en utilisant la matière même de la coque 3 thermoformable mais dans une quantité extrêmement réduite en comparaison des techniques de surmoulage totale enseignées dans l'art antérieur. On réduit ainsi considérablement le coût matière dans la réalisation de la gouttière dentaire de l'invention, de même que les temps de fabrication.

Par ailleurs, la réduction de matière nécessaire au surmoulage de l'armature 2 procure une réduction importante de l'encombrement total de la gouttière dentaire 1 une fois constituée, ce qui procure un confort d'utilisation accru pour les patients.

Comme représenté en particulier sur les figures 5 et 7, la gouttière dentaire 1 de l'invention comporte avantageusement en outre en guise de moyens d'ancrage des orifices 5 formés de part en part dans la paroi occlusale 23 de l'armature 2, notamment de préférence à proximité des extrémités postérieures de chacune des branches latérales 2a, 2b de l'armature 2. Ces orifices 5 sont conformés pour permettre, lors de la formation de la coque 3 thermoformable sur l'armature 2 et/ou lors de la mise en forme par prise d'empreinte d'une arcade dentaire d'un patient dans la coque 3 thermoformable de la gouttière dentaire 1 de l'invention , une insertion de la matière thermoformable de la coque 3 au travers des orifices 5 depuis la surface interne de la paroi occlusale 23 vers la surface externe de cette même paroi occlusale 23, formant ainsi des tenons d'ancrage de la coque 3 sur l'armature 2. Les orifices 5 sont en outre conformés pour empêcher le retrait de la matière thermoformable de la coque 3 de la surface externe vers la surface interne de la paroi occlusale 23. De cette manière, la coque 3 thermoformable est effectivement ancrée sur la paroi occlusale 23 de l'armature 2 par l'intermédiaire de tenons d'ancrage en matière thermoformable dans les orifices 5, comme cela apparait en particulier sur la figure 7.

Plus précisément et de façon avantageuse selon l'invention les orifices 5 sont tels qu'ils présentent au moins une dimension, prise dans au moins une section verticale de la paroi occlusale 23 comme représenté sur la figure 7, qui est inférieure au niveau de la surface interne de la paroi occlusale 23 par rapport à la dimension correspondante du même orifice au niveau de la surface externe de la paroi occlusale 23. De préférence, la dimension des orifices 5 est ainsi variable de manière croissante sur l'épaisseur de la paroi occlusale 23 entre la surface interne et la surface externe de ladite paroi occlusale 23. Selon ce principe de réalisation préféré, les orifices 5 ont une section, prise dans le dit plan vertical, en forme de queue d'aronde, c'est-à-dire de forme au moins partiellement triangulaire ou trapézoïdale. Alternativement une section en T peut également être considérée.

Les moyens d'ancrage de la coque thermoformable 3 de la gouttière dentaire 1 de l'invention procurent une liaison performante de ces deux éléments empêchant tout déchaussement de la coque thermoformable 3 de l'armature 2 de la gouttière 1 même en cas d'utilisation prolongée de celle-ci et de contraintes mécaniques de mastication fortes de la part de patients, tout en permettant comme précédemment évoqué une réduction de matière importante de la coque thermoformable 3 et donc de l'encombrement global de la gouttière dentaire 1, favorisant un meilleur confort d'utilisation pour le patient avec notamment moins de lésions des tissus buccaux au contact de la gouttière dentaire 1 en comparaison des gouttières connues de l'art antérieur.

Afin d'améliorer toujours le confort et la polyvalence d'utilisation pour les patients l'armature 2 de la gouttière dentaire 1 de l'invention est avantageusement configurée pour être flexible de part et d'autre d'au moins un plan vertical, notamment au moins un plan sagittal médian M de la gouttière 1 (figures 1, 4, 5), perpendiculaire à la paroi occlusale 23 de l'armature 2. De préférence encore, l'armature 2 est plus largement flexible de part et d'autre d'une pluralité de plans verticaux P1, P2, P3, P4, P5, P6 perpendiculaires de la paroi occlusale 23 de l'armature 2 comme représenté sur la figure 5.

Afin de conférer un caractère flexible à l'armature par rapport à un ou plusieurs plan verticaux comme précédemment décrit, ladite armature 2 comporte au moins une zone de fléchissement privilégiée formée par réduction localisée de la matière constitutive de l'armature 2, laquelle s'étend par exemple dans au moins un dit plan vertical de part et d'autre duquel l'armature 2 est flexible.

Dans une forme avantageuse de réalisation, la ou les zones de fléchissement privilégiée(s) de l'armature 2 résulte(nt) d'un évidement 6, en particulier une fente, formée dans au moins une paroi labiale ou linguale et la paroi occlusale de l'armature. De préférence, le ou les dits évidements 6 sont formés exclusivement dans la paroi linguale 22 et la paroi occlusale 23 de l'armature 2.

Dans un mode de réalisation préféré tel que représenté sur la figure 5 l'armature 2 comporte plusieurs zones de fléchissement formées par des évidements 6 ou retraits de matière localisés dans ses parois linguale 22 et occlusale 23. Cette forme de réalisation procure le meilleur équilibre entre flexibilité et rigidité de l'armature 2 afin de procurer à la fois une souplesse d'ajustement à tout type de conformation d'une arcade dentaire d'un patient tout en procurant une gouttière dentaire 1 stable et dotée d'un bon maintien élastique sur l'arcade dentaire du patient.

Une autre caractéristique avantageuse mais accessoire de la gouttière dentaire 1 de l'invention telle que représentée aux figures 2 à 7 en particulier réside dans l'intégration, dans la face externe de la paroi labiale 21 de l'armature 2 et dans le plan médian M de la gouttière 1 d'un téton ou plot 7 saillant comprenant une tête 71 et un pied 72 de liaison à la paroi labiale 21.

Ce téton 7 procure une triple fonctionnalité additionnelle à la gouttière dentaire 1 de l'invention comme décrit ci-après en référence aux figures 8 à 12.

Comme représenté sur la figure 8 tout d'abord, le téton 7 de la gouttière 1 forme un organe de blocage pour positionner la gouttière 1 sur une plaque occlusale 9 pour réaliser une prise d'empreinte dentaire et la mise en forme de la gouttière 1. Par souci de clarté, seule l'armature 2 de la gouttière dentaire 1 est représentée sur la figure 8 afin de montrer le positionnement particulier de la gouttière sur la plaque occlusale 9.

La plaque occlusale 9, avantageusement procurée avec la gouttière 1 de l'invention pour former un kit permettant à tout patient de mettre en forme la gouttière 1 en ambulatoire chez lui, présente une forme en Y dont le pied forme une branche 9a de saisie manuelle prolongée de deux branches de support 9b, 9c jointes à une de leur extrémités en V formant un angle aigue à leur base. Ces deux branches en V constituent des plans de support de la gouttière dentaire 1. Sur les branches de support 9b, 9c sont en outre positionnés, ou préférentiellement formés (par exemple par moulage de la plaque occlusale 9) des plots de calage 9d saillants sur les branches de support 9b, 9c de ladite gouttière 1. Les plots de calage 9d, de préférence au nombre de 4 au minimum, sont agencés de manière à caler la gouttière 1 en position sur les branches de support 9b, 9c de la plaque occlusale 9, notamment par appui des sections latérales 2a, 2b de l'armature 2 de la gouttière dentaire 1 contre au moins un plot 9d chacune sur chaque branche de support 9b, 9c respectivement et, au niveau de la section centrale 2c de l'armature 2 de la gouttière 1 par insertion du téton 7 de l'armature 2 entre deux plots de calage 9d espacés l'un de l'autre d'une distance sensiblement également au diamètre du pied 72 du téton 7 dans un plan perpendiculaire à l'axe longitudinal dudit pied 72, lequel s'étend dans le plan sagittal médian M de la gouttière 1. Le téton 7 en coopération des plots de calage 9d de la plaque occlusale 9 permet un blocage stable de la gouttière 1 sur la plaque occlusale 9 pour réaliser la mise en forme de la gouttière dentaire selon un procédé décrit ci-après.

Pour ce faire, un patient doit simplement procéder aux étapes suivantes.

Dans un premier temps, il convient de procéder au chauffage de la gouttière dentaire 1, par exemple par plongée de la gouttière 1 intégralement dans un bain-marie à une température comprise entre 60 et 80°C pour ramollir la coque 3 de matière thermoformable, au minimum pendant une durée de 1 min au minimum, de préférence une durée de 2 à 5 min.

Ensuite, on positionne la gouttière dentaire 1 sur la plaque occlusale 9 en prenant soin de caler celle-ci par insertion du téton 7 entre les plots de calage 9d de manière à stabiliser la gouttière dentaire 1 pour la prise d'empreinte.

Dans une troisième étape le patient insert ensuite la plaque occlusale 9 portant la gouttière dentaire 1 dont la coque thermoformable 3 est ramollie dans sa bouche puis procède à la prise d'empreinte et mise en forme de la coque thermoformable 3 et de l'armature 2 de la gouttière dentaire 1 par compression de la coque thermoformable 3 entre une arcade dentaire et la plaque occlusale 9 par serrage de la mandibule contre la plaque occlusale 9. De préférence, on veillera à maintenir l'effort de compression pendant au moins 20 secondes à une minutes puis, on peut ensuite retirer la plaque occlusale 9 et de la gouttière dentaire 1 mise en forme de la bouche du patient. Le cas échéant, une étape additionnelle de refroidissement de la gouttière peut être envisagée pour figer parfaitement la forme d'empreinte réalisée, ainsi que faciliter l'élimination ultérieure de bavures éventuelles afin de permettre un ajustement parfait dans la bouche du patient lors de l'utilisation de la gouttière.

Lors de la mise en forme de la gouttière dentaire 1 la plaque occlusale permet avantageusement d'éviter les fuites de la matière thermoformable constitutive de la coque 3 vers l'extérieur de l'armature 2 au travers d'orifices 5 et des évidements ou fentes 6 de ladite armature. On évite ainsi un accroissement volumique de la gouttière dentaire 1 lors de la prise d'empreinte. En outre, la contrepression réalisée par la plaque occlusale 9 lors de la prise d'empreinte favorise avantageusement la formation des tenons d'ancrage de la coque 3 dans les orifices 5 prévus à cet effet dans l'armature 2 en plaquant la matière de la coque thermoformable 3 s'écoulant au travers de ces orifices 5 lors la prise d'empreinte, favorisant la répartition de la matière dans les orifices, notamment dans les sections de plus grandes dimensions, ce qui évite ensuite le retrait de matière dans ces orifices 5 lors du refroidissement de la coque 3 de matière thermoformable.

Une autre fonction du téton 7 de l'armature 2 de la gouttière dentaire 1 consiste en outre à fournir une référence centrée et visible sur la gouttière 1 pour faciliter le centrage de celle-ci lors de l'insertion en bouche du patient pour la prise d'empreinte. Ceci peut s'avérer avantageux dans le cas d'asymétrie dentaire par rapport au plan anatomique sagittal médian d'un patient.

Le téton 7 procure enfin une dernière fonctionnalité accessoire à la gouttière 1 de la présente invention, qui consiste à permettre la fixation d'une bande de liaison de deux gouttières dentaires dans le cadre de la réalisation d'une orthèse mandibulaire comme décrit ci-après en référence aux figures 9 à 12.

Les orthèses mandibulaires sont classiquement prescrites et usitées dans le cadre d'un traitement de l'apnée du sommeil et comportent au moins une gouttière dentaire mandibulaire et un système de rappel élastique de cette gouttière mandibulaire vers l'avant afin d'éviter les glissements postérieur de la mandibule durant le sommeil, cause des pauses respiratoires typiques de l'apnée du sommeil. Le système de rappel élastique est classiquement constitué d'un bandeau de matière élastique solidaire à ses extrémités de points d'ancrage postérieurs sur la gouttière mandibulaire, le bandeau étant mis en tension dans la bouche du patient contre l'arcade maxillaire supérieur du patient.

Afin d'améliorer le confort des patients utilisant de telles orthèses mandibulaires il est préférable que l'orthèse comporte non pas une mais deux gouttières, une gouttière mandibulaire et une gouttière dentaire de type occlusal pour le maxillaire supérieur, les deux gouttières étant alors reliées entre elles par un bandeau élastique pour exercer une force de propulsion sur la mandibule, tout en permettant une certaine liberté de mouvement.

Une orthèse mandibulaire 100 comportant deux gouttières dentaires 101, 102 conformes à la présente invention reliées entre elles par un bandeau élastique 103 est représentée selon différentes vues aux figures 9 à 12.

La gouttière dentaire supérieure 101 de l'orthèse mandibulaire 100 est identique à la gouttière des figures 1 à 7. La gouttière dentaire inférieure 102, ou gouttière mandibulaire diffère de la gouttière dentaire supérieure 101 uniquement par l'absence de téton 7 frontal. En revanche la gouttière 103 comporte deux tétons latéraux 8 solidaires et saillants sur l'armature de la gouttière 103. Ces tétons latéraux 8 permettent la fixation et le retrait des extrémités du bandeau de liaison 103, lesquelles comportent avantageusement chacune des orifices prévus à cet effet. Le bandeau 103 est en outre fixé par un orifice également prévu à cet effet sensiblement en son milieu sur le téton 7 de la gouttière supérieure 101. Ainsi obtient-on un effet de bandage élastique des deux gouttières 101, 102 l'une par rapport à l'autre de sorte que le bandeau 103 exerce, lorsque les deux gouttières 101, 102 sont positionnées sur les maxillaires supérieur et inférieur respectivement d'un patient, un effort de traction postéro-antérieur de la mandibule afin d'éviter tout glissement postérieur de celle-ci lors des phases de sommeil du patient afin de prévenir l'apnée du sommeil.

Lors de l'usage, les surfaces extérieures des parois occlusales des armatures de chacune des gouttières 101, 102 se trouvent en contact le long du plan d'occlusion, et peuvent glisser l'une sur l'autre.

La présente invention propose donc une gouttière dentaire d'encombrement réduit et de confort amélioré adaptée aux traitements du bruxisme et/ou de l'apnée du sommeil que ce soit en utilisation simple avec une seule gouttière dentaire conformée selon le procédé de mise en forme de l'invention pour un des maxillaires d'un patient ou dans le cadre d'une orthèse mandibulaire comportant une ou deux gouttières dentaires conformes à la présente invention reliées entre elles par un bandeau de liaison élastique.

## Revendications

1. Gouttière dentaire (1), comprenant une armature (2) d'une matière synthétique rigide supportant une coque (3) de matière thermoformable, ladite armature (2) comportant une paroi antérieure, dite paroi labiale (21), et une paroi postérieure, dite paroi linguale (22), sensiblement parallèles l'une de l'autre et reliées par une paroi de fond, dite paroi occlusale (23), sensiblement perpendiculaire aux deux parois labiale (21) et linguale (22) de sorte que l'armature (2) présente dans tout plan vertical une section en U, **caractérisée en ce que** ladite coque thermoformable (3) est logée entre lesdites parois labiale (21), linguale (22) et occlusale (23) de ladite armature essentiellement et ancrée sur celle-ci par l'intermédiaire de moyens d'ancrage (4, 5) agencés dans l'armature (2) et la coque thermoformable (3).

2. Gouttière dentaire (1) selon la revendication 1, **caractérisée en ce que** les moyens d'ancrage comportent au moins une patte de liaison (4) recouvrant au moins partiellement au moins un segment de la paroi labiale (21) et/ou de la paroi linguale (22), ladite patte de liaison (4) étant formée de matière avec la coque thermoformable (3) de manière à pincer ledit au moins un segment de paroi labiale et/ou linguale entre la coque thermoformable (3) et la patte de liaison (4).

3. Gouttière dentaire (1) selon la revendication 1 ou 2, **caractérisée en ce que** les moyens d'ancrage comportent des orifices (5) formés de part en part entre une surface interne de la paroi occlusale (23) sur laquelle repose la coque thermoformable (3) et une surface externe de la paroi occlusale (23), lesdits orifices (5) présentant au moins une dimension, prise dans au moins une section verticale de la paroi occlusale, qui est inférieure au niveau de la surface interne par rapport à la dimension correspondante au niveau de la surface externe.

4. Gouttière dentaire selon la revendication 3, **caractérisée en ce que** les dits orifices (5) présentent une dimension variable de manière croissante sur l'épaisseur de la paroi occlusale entre la surface interne et la surface externe de ladite paroi occlusale (23).

5. Gouttière dentaire selon l'une des revendications 3 ou 4, **caractérisée en ce que** lesdits orifices (5) ont une section, prise dans ladite section verticale de la paroi occlusale, au moins partiellement triangulaire ou trapézoïdale.

6. Gouttière dentaire selon l'une des revendications 1 à 5, caractérisée en ce ladite armature (2) est flexible de part et d'autre d'au moins un plan vertical (M) perpendiculaire à sa paroi occlusale (23).

7. Gouttière dentaire selon l'un des revendications 1 à 6, **caractérisée en ce que** ladite armature est flexible de part et d'autre d'une pluralité de plans verticaux (P1, P2, P3, P4, M) perpendiculaires à sa paroi occlusale (23).

8. Gouttière dentaire selon l'une des revendications 1 à 7, **caractérisée en ce que** ladite armature (2) comporte au moins une zone de fléchissement privilégiée formée par réduction localisée de la matière constitutive de l'armature.

9. Gouttière dentaire selon les revendications 6 à 8, **caractérisée en ce que** ladite au moins une zone de fléchissement privilégiée s'étend dans au moins un dit plan vertical (P1, P2, P3, P4, P5, P6) de part et d'autre duquel ladite armature (2) est flexible.

10. Gouttière dentaire selon l'une des revendications 8 ou 9, **caractérisée en ce que** la au moins une zone de fléchissement privilégiée comporte un évidement (6), en particulier une fente, formée dans au moins une paroi labiale (21) ou linguale (22) et la paroi occlusale (23) de l'armature.

11. Gouttière dentaire selon l'une des revendications 8 ou 9, **caractérisée en ce que** la au moins une zone de fléchissement privilégiée comporte un évidement (6) formé exclusivement dans la paroi linguale (22) et la paroi occlusale (23).

12. Gouttière dentaire selon l'une des revendications 8 à 11, **caractérisée en ce qu'**elle comporte plusieurs zones de fléchissement formées par des évidements (6) ou retraits de matière localisés dans les parois linguale (22) et occlusale (23).

13. Procédé de mise en forme d'une gouttière dentaire (1) selon l'une des revendications 1 à 12, comprenant les étapes suivantes :
- Chauffage de la gouttière dentaire (1) dans un bain-marie à une température comprise entre 60°C et 90°C pour ramollir la coque (3) de matière thermoformable pendant une durée de 1 min au moins;
- Positionnement de la gouttière dentaire sur une plaque occlusale (9) conformée sensiblement en Y, la plaque occlusale (9) comportant une branche de saisie manuelle (9a) et deux branches de support (9b, 9c) de la gouttière dentaire jointes à une de leurs extrémités et à une extrémité de la branche de saisie manuelle ainsi que des plots de calage (9d) de ladite gouttière dentaire (1) ajustés sur une face de ladite plaque occlusale au moins ;
- Insertion de la plaque occlusale portant la gouttière dentaire dans la bouche d'un patient,
- Mise en forme de la coque (3) de matière thermoformable par pression d'une arcade dentaire du patient dans la coque (3) ramollie et contrepression sur la plaque occlusale (9) de l'arcade dentaire opposée de manière à procurer un effet de coffrage de la matière ramollie de la coque dans l'armature (2) ;
- Retrait de la plaque occlusale (9) et de la gouttière dentaire (1) de la bouche du patient.

14. Procédé selon la revendication 13, comportant une étape additionnelle de refroidissement de la gouttière dentaire (1) après retrait de la plaque occlusale (9) de la bouche du patient.

15. Orthèse mandibulaire (100) comportant au moins une gouttière dentaire (101, 102) telle que définie à l'une des revendications 1 à 12.
